# EUROPEAN PATENT SPECIFICATION

(11) **EP 1 393 725 B1**
(45) Date of publication and mention of the grant of the patent: **21.04.2010**
(21) Application number: 01963497.1
(22) Date of filing: 07.09.2001
(51) Int. Cl.: A61K 31/198, A61P 25/00

(54) **COMPOSITIONS FOR AMELIORATING ATTENTION-DEFICIENT/HYPERACTIVITY DISORDER**
ZUSAMMENSETZUNGEN ZUR VERBESSERUNG VON ATTENTION DEFICIENT HYPERACTIVITY DISORDER (ADHD)
COMPOSITIONS DESTINEES A AMELIORER LES TROUBLES DE MANQUE D'ATTENTION/D'HYPERACTIVITE

(30) Priority: 06.06.2001 JP 2001171342
(43) Date of publication of application: 03.03.2004
(73) Proprietor: TAIYO KAGAKU Co., LTD., Yokkaichi-shi Mie-ken 510-0825 (JP)
(72) Inventor: KUMAGAI, Tomoko, Yokkaichi-shi, Mie 510-0825 (JP); OZEKI, Makoto, Yokkaichi-shi, Mie 510-0825 (JP); OKUBO, Tsutomu, Yokkaichi-shi, Mie 510-0825 (JP); JUNEJA, Lekh Raj, Yokkaichi-shi, Mie 510-0825 (JP)
(74) Representative: Schüssler, Andrea
(86) International application number: PCT/JP2001/007763
(87) International publication number: WO 2002/100393

(56) References cited:
- EP-A- 1 057 483
- EP-A2- 1 074 252
- WO-A1-99/04785
- JP-A- 9 012 454
- JP-A- 9 040 568
- MASON R: "200 mg of Zen: L-Theanine boosts alpha waves, promotes alert relaxation" ALTERNATIVE AND COMPLEMENTARY THERAPIES 2001 UNITED STATES, vol. 7, no. 2, April 2001 (2001-04), pages 91-95, XP009059302 ISSN: 1076-2809
- JUNEJA L R ET AL: "L-THEANINE-A UNIQUE AMINO ACID OF GREEN TEA AND ITS RELAXATION EFFECT IN HUMANS" TRENDS IN FOOD SCIENCE AND TECHNOLOGY, ELSEVIER SCIENCE PUBLISHERS, GB, vol. 10, no. 12, 1999, pages 199-204, XP002959796 ISSN: 0924-2244
- DATABASE WPI Section Ch, Week 199621 Derwent Publications Ltd., London, GB; Class B05, AN 1996-205436 XP002182609 & JP 08 073350 A (ITOEN KK) 19 March 1996 (1996-03-19)

## Description

### TECHNICAL FIELD

The present invention relates to a composition for use in ameliorating attention-deficit hyperactivity disorder, and its used for manufacturing a medicament for ameliorating attention-deficit hyperactivity disorder.

### BACKGROUND ART

Attention-deficit hyperactivity disorder (ADHD) is neurological disorders in which an individual cannot control concentration, attentiveness, impulsive characteristic or hyperactivity by himself. The attention-deficit hyperactivity disorder has been considered as one of disorders of which incidence is found in little children. However, recently, psychiatrists have elucidated that the disorder is not only found in childhood but also maintained in adults. A feature of this disorder resides in accompaniment of three symptoms, attention disorder, activity disorder and abnormal impulsive characteristic, and the disorder is distinguished from a wide-spread developmental disorder such as autism, manic depression or anxiety disorder. Also, among the attention-deficit hyperactivity disorders, those that tend not to highly show hyperactivity (over-activity) are referred to as attention-deficit disorder (ADD), and those that tend not to highly show attention deficit (lowering of attentiveness) are referred to as hyperactivity disorder.

As the causations for the attention-deficit hyperactivity disorders, there have been recited various factors which may bring about cranial damages or abnormal development, such as injuries, diseases, influence of alcohol and cigarette intake during embryonic stage, and influence of high-concentration lead during an earlier stage of development, heredity, secretion, re-uptake and abnormal degradation of neurotransmitters, inactivation of actions of specified region of the brain, and the like. However, an accurate causation has not yet been known.

Conventionally, rehabilitational training and pharmacotherapy have been mainly employed for the treatment of this disorder. As the rehabilitational training, a patient is subjected to psychotherapy, behavioral therapy, sensory integration therapy or the like, to train the patient so that an appropriate social life can be conducted. However, such a therapy does not have an immediate effect and would necessitate cooperation of home and school, and an enormous amount of time and specialized education would be required for the therapy. On the other hand, drugs tend to bring about many side effects, so that careful management and dosage would be necessary. For instance, there are tomoxetine (Japanese Unexamined Publication No. Hei 10-512262), 2-(4-methylaminobutoxy)diphenylmethane (Japanese Patent Laid-Open No. Hei 8-81366) and the like, which require dosage. Also, pycnogenol (extract from bark of pine in French coastal area) has been reported to have an effect for ameliorating ADHD. However, since this drug is very expensive, it cannot be easily used.

An object of the present invention is to provide a composition for the use in ameliorating attention-deficit hyperactivity disorder which has no side effects, is inexpensive, and can be used on a daily basis; and a method of effectively ameliorating attention-deficit hyperactivity disorder.

### DISCLOSURE OF INVENTION

As a result of intensive studies in order to solve the above-mentioned problems, the present inventors have found that the desired effects can be exhibited by theanine contained in green tea. The present invention has been perfected thereby.

Specifically, the present invention relates to:
[1] Use of theanine for manufacturing a medicament for ameliorating attention-deficit hyperactivity disorder.
[2] Theanine for use in ameliorating attention-deficit hyperactivity disorder.

Further aspects of the present invention are mentioned in dependent claims 2-6, 8 and 9.

### BEST MODE FOR CARRYING OUT THE INVENTION

The composition for use in ameliorating attention-deficit hyperactivity disorder (hereinafter referred to as "the composition") of the present invention is characterized in that the composition comprises theanine. According to the composition, the attentive-deficit hyperactivity disorder can be effectively ameliorated. Also, the composition has no risks of side effects, and is inexpensive, so that the composition can be used on a daily basis. The desired effects of the composition of the present invention are exhibited due to the actions of theanine contained in the composition for use in mitigating and alleviating the attention-deficit hyperactivity disorder. The actions have been found for the first time on theanine.

The "attention-deficit hyperactivity disorder," as referred to herein, to which the composition of the present invention is applied is a symptom judged on the basis of a diagnostic method for ADHD, as defined in DSM-IV Seishin Shogai no Shindan •Tokei Manyuaru (Diagnosis and Statistic Manuals for DSM-IV Mental Disorders) (published by Igakushoin, 1996). Concretely, an individual with ADHD meets the following criteria.

### [Criteria for Diagnosis]

An individual is judged to be attention-deficit hyperactivity disorder when showing 6 or more symptoms among the following list of symptoms of "Lack of Attention," or 6 or more symptoms among the following list of symptoms of "Hyperactivity and Impulsive Characteristic." However, the judgement for attention-deficit hyperactivity disorder is made under the conditions that these symptoms are frequently seen, and there have been maintained for at least 6 months inappropriate behaviors which do not correspond to the development level of a patient. In addition, the diagnosis is made under the conditions that some of these symptoms have caused problems before reaching 7-years old, and that any problems take place at present in two or more different locations (examples: school and home). Also, the judgment is made only in the case where serious disorders are recognized in social life, schoolwork and vocational work, and any of the symptoms are not caused by other diseases.

### (Symptoms of Lack of Attention)

- not being able to carefully pay attention to things; making mistakes due to lack of attention in schoolwork, vocational work or other activities.
- finding it difficult to maintain attention to assigned job or play.
- not seeming to be listening even when directly spoken.
- not being able to follow through an instruction; not being able to carry out duties in schoolwork, daily miscellaneous trifles, or vocational work.
- not being able to orderly carry out assigned job or activity.
- avoiding or disliking to perform vocational or schoolwork that requires to maintain mental activities, or performing it unwillingly.
- losing things that are required for vocational work or activity (toys, homework for school, pencils, books, tools and the like).
- attention being distracted by stimuli from others.
- forgetting daily activities.

### (Symptoms for Hyperactivity and Impulsive Characteristic)

- busily moving hands or fidgetingly sitting.
- leaving one's seat or standing up in a classroom in a situation where one should not.
- running around or climbing over a certain object in an inappropriate situation (having an emotion of restlessness when reaching adolescence or adult).
- not being able to spend free time on quietly playing or the like.
- constantly being active, moving about as if "driven by a motor."
- over-talking.
- suddenly answering before a question is finished.
- not being able to wait in order.
- disturbing others.

Here, the phrase "ameliorating attention-deficit hyperactivity disorder" refers to, for instance, mitigating or alleviating symptoms of attention-deficit hyperactivity disorder, which in wide sense includes the prevention or treatment of attention-deficit hyperactivity disorder.

The exhibition of the action for mitigating or alleviating attention-deficit hyperactivity disorder by theanine can be evaluated by a clinical test in which theanine is administered to a patient who meets the above-mentioned [Criteria for Diagnosis], and any ameliorative effects on attention-deficit hyperactivity disorder are observed, as compared to that before the administration of theanine.

Theanine used in the present invention is a glutamic acid derivative (γ-glutamylethylamide), which is an amino acid component naturally contained largely in tea-leaves. Methods for preparing theanine used in the present invention include, for instance, a method of extracting from tea-leaves; an organic synthesis method [Chem. Pharm. Bull., 19(7), 1301-1307 (1971)]; a method of treating a mixture of glutamine and ethylamine with glutaminase (Japanese Unexamined Patent Publication No. Hei 7-55154); a method comprising culturing cultured cells of tea in a medium containing ethylamine, thereby achieving growth promotion of the cultured cells while increasing the cumulative amount of theanine in the cultured cells (Japanese Patent Laid-Open No. Hei 5-123166); modification methods in which ethylamine is substituted by an ethylamine derivative such as ethylamine hydrochloride in the methods disclosed in Japanese Unexamined Patent Publication No. Hei 7-55154 or Japanese Patent Laid-Open No. Hei 5-123166; and the like, and any of the methods may be used. The above-mentioned "tea-leaves" include green tea-leaves, oolong tea-leaves, black tea-leaves, and the like.

Theanine can be used as any of L-theanine, D-theanine and DL-theanine. Among them, the L-form is preferred in the present invention, because the L-form is approved as a food additive, and is economically utilizable. In addition, theanine used in the present invention may be in any forms, such as purified products, crudely purified products and extracts. Also, a commercially available product [SUNTHEANINE (registered trade mark), manufactured by Taiyo Kagaku Co., Ltd.] may be used.

The content of theanine in the composition of the present invention is not particularly limited, and the content may be appropriately adjusted as desired. For example, the preferred content of theanine in the composition is preferably from 0.00025 to 100% by weight, more preferably from 0.001 to 100% by weight, still more preferably from 0.001 to 20% by weight in consideration of the exhibition of the desired effects of the present invention.

A method of detecting theanine in the composition of the present invention is not particularly limited. It is preferable that the method comprises derivatizing by orthophthalaldehyde (OPA) in pre-column, separating by high-performance liquid chromatography (HPLC) using ODS column, and detecting and quantifying with a fluorescence detector, or that the method comprises separating by HPLC using ODS column, and detecting and quantifying at a wavelength of 210 nm.

The composition of the present invention may further contain various minerals. The composition comprising a mineral is more preferable, because there is exhibited such an effect that the composition can supply a living body with essential elements and trace essential elements, which tend to be deficient in living bodies. The mineral content in the composition, for example, is preferably from 0.0001 to 99.9% by weight and more preferably from 0.01 to 99.9% by weight. The mineral includes metals or metal salts essential for maintaining and regulating homeostasis of living bodies, such as iron, magnesium, copper, zinc, selenium, calcium, potassium, manganese, chromium, iodine, molybdenum, nickel and vanadium, or metal salts thereof. These minerals can be used alone or in admixture of two or more kinds.

Also, crude medicines, herbs, amino acids, vitamins, and other materials and raw materials which are acceptable in foods may also be contained. These can be used alone or in admixture of two or more kinds.

The crude medicines include, but are not particularly limited to, for instance, *Gymnema sylvestra, Garcinia cambogia*, *Common valerian, Chinese guta percha, Angelica acutiloba, Paeonia lactiflora*, peony, *Panax ginnseng,* reisi (*ganoderma*), rehmannia root, common jujube and *Acanthopanax senticosus harms*, and reisi (*ganoderma*), rehmannia root, and common jujube which are effective in stabilizing mind-conditions are preferable. The forms of the crude medicines may be, but are not limited to, any of extracts, dry products and the like. The herbs include, but are not limited to, *Amla,* anise, carrot seed, clove, coriander, cypress, cinnamon, juniper, ginger, sweet orange, pine needle, basil, patchouli, bitter orange, fennel, black pepper, bay, peppermint, bergamot, mandarin, myrrh, lemongrass, rosemary, grapefruit, cedarwood, citronella, sage, thyme, tea tree, violet leaf, vanilla, hyssop, eucalyptus, lime, lemon, ylang-ylang, cardamon, clary sage, jasmine, geranium, chamomile, Bulgarian rose, rose, olibanum, lavender, chamomile, geranium, sandalwood neroli, verbena, petigrain, vetiver, majoram, lemon balm (*Melissa officinalis*), rosewood, *Hypericum*, St. John's wort, kawakawa, passion flower and *Black cohosh,* among which preference is given to peppermint, bergamot, ylang-ylang, geranium, chamomile, lavender, St. John's wort, and kawakawa, which have sedative and relaxation effects. The forms of these herbs include, but are not particularly limited to, extracts, essential oils, herb teas and the like. The amino acid includes, but are also not particularly limited to, for example, L-form amino acids such as alanine, arginine, arginine acetate, arginine hydrochloride, asparagine, aspartic acid, citrulline, cysteine, cystine, glutamine, glutamic acid and a salt thereof, glycine, histidine and a salt thereof, hydroxyproline, isoleucine, leucine, lysine and a salt thereof, methionine, ornithine acetate and ornithine hydrochloride, phenylalanine, proline, serine, threonine, tryptophan, tyrosine and valine; DL-form amino acids such as alanine, cysteine and a salt thereof, methionine, phenylalanine, threonine, tryptophan and valine; D-form amino acids such as alanine, cysteine hydrochloride hydrate and phenylalanine; and the like. Also, there are included composite salts and mixtures of L-amino acids such as those of L-arginine and L-aspargine; a metal salt of an amino acid such as potassium aspartate; an ester of an amino acid such as L-ethylcysteine hydrochloride; an acetylamino acid such as acetylcysteine; nucleic acid-associated substances such as adenine and adenosine; omega-amino acid such as β-alanine; an amino acid metabolite such as histamine dihydrochloride; γ-aminobutyric acid, taurine, thiotaurine and hypotaurine. The vitamin includes, but is not particularly limited to, for example, vitamin A, vitamin B1, vitamin B2, vitamin B6, vitamin B12, vitamin C, vitamin D, vitamin E, vitamin K, folic acid, nicotinic acid, lipoic acid, pantothenic acid, biotin, ubiquinone and prostaglandin, as well as derivatives of these vitamins. The other materials and raw materials which are acceptable in foods include, for example, *Lepidium meyenii,* cat's-claw, squalane, *Phellinus linteus Aoshima*, DHA, EPA, ceramide, lactoferrin, pycnogenol, aloe, royal jelly, melatonin, placenta, propolis, isoflavone, soybean lecithin, egg yolk lecithin, egg yolk oil, chondroitin, cacao mass, collagen, vinegar, chlorella, spirulina, gingko leaf, green tea, tochu tea (*Eucommia ulmoides*), Chinese wolfberry tea, oolong tea, mulberry leaf, Rubus suavissimus (tencha), banaba tea; functional materials such as unsaturated fatty acids, saccharides such as oligosaccharides, dietary sweeteners, dietary fibers and soybean peptides; microorganisms such as bifidobacteria and red koji; mushrooms such as agaricus (*Agaricus blazei*), Hime Grifloa frondosa and Grifloa frondosa; fruits such as blueberry, prune, grape, olive, Japanese apricot, and citruses; seeds such as peanut, almond, sesame, and pepper; vegetables such as green pepper, chili, Welsh onion, pumpkin, gourd, carrot, burdock, jute leaf (*Corchorus capsularis*), garlic, perilla, wasabi, tomato, pickled shallot, ginger, leaf vegetables, tubers, and beans; seaweeds such as wakame; fish; meat, poultry, and whale meat; and cereals; as well as their extracts, dried products, crudely purified products, purified products, processed products, and fermented products and the like. Among them, functional materials such as dietary sweeteners, dietary fibers and soybean peptides are preferable.

In addition, as the composition of the present invention, a food composition or a pharmaceutical composition is preferable, from the viewpoint of its suitability for the daily use.

The food composition according to the present invention encompasses not only a food comprising theanine but also a food additive comprising theanine.

The above-mentioned food according to the present invention includes a solid food such as dry foods and supplements, and a liquid food such as soft drinks, mineral water, luxury beverages and alcoholic beverages. Specifically, the solid food includes, but is not particularly limited to, paste products, processed soy products, mousse, jelly, yogurt, cold confectioneries, candies, chocolates, gum, crackers, biscuits, cookies, cake, bread and the like. In addition, the liquid food includes, but is not particularly limited to, teas such as green tea, oolong tea, black tea and herb tea, fruit juice concentrates, reconstituted juice concentrates, fresh juices, mixed fruit juices, fruit grain-containing fruit juice, fruit juice-containing beverages, mixed fruit/vegetable juice, vegetable juice, carbonated beverages, soft drinks, milk, milk beverage, Japanese *sake,* beer, wine, cocktails, *shochu,* whiskey, and the like.

In addition, the pharmaceutical composition of the present invention is not particularly limited as long as the pharmaceutical composition comprises theanine. For instance, the pharmaceutical composition may be any of solutions, suspensions, powders, solid molded products and the like. Therefore, the preparation forms of the pharmaceutical composition include tablets, capsules, powdered agents, granules, health care drinks and the like. The pharmaceutical composition can also be used in combination with other medicaments.

Other medicaments include, but are not particularly limited to, for instance, psychotropic drugs such as those medicaments which are used in ADHD treatment, such as methylphenidate hydrochloride, pemoline, imipramine hydrochloride, maprotiline hydrochloride, nortriptypline hydrochloride, and 2-(4-methylaminobutoxy)diphenylmethane; agents for inhibiting norepinephrine absorption such as tomoxetine; antihypertensive agents, antipsychotic agents, anti-mania agents, antianxiety agents, narcoleptic agents, psychostimulants, antiepileptic agents, sedatives, anti-Parkinson agents, agents for inhibiting selective serotonin resorption, and antidepressants such as agents for inhibiting selective serotonin adrenaline resorption; Chinese herb medicines such as Kamishousou san and Touki-shakuyaku san.

A process for preparing the composition of the present invention is not particularly limited, and there can be used general processes for preparation of a food or a medicament such as a process of powder-mixing theanine and other raw materials; a process of dissolving theanine and other raw materials in a solvent to give a mixed solution; and a process of freeze-drying the mixed solution; a process of spray-drying the mixed solution. The components other than theanine which can be used when the composition of the present invention is prepared can be selected appropriately in accordance with the desired use, so long as the exhibition of the desired effects by the theanine is not inhibited.

For instance, the food composition of the present invention can be prepared by adding theanine to the conventional food by a conventional method so that the content of theanine in the food composition of the present invention after the preparation is preferably within the above-mentioned range of the preferred content of theanine in the composition. In addition, the pharmaceutical composition of the present invention can be prepared by formulating theanine by a conventional method, together with, for instance, a known organic or inorganic vehicle suitable for oral administration, excipient, binder, stabilizing agent, and the like, so that the content of theanine in the pharmaceutical composition of the present invention is preferably within the above-mentioned range of the preferred content of theanine in the composition as in the case of preparation of the food composition. Accordingly, use of theanine for manufacturing the medicament for ameliorating attention-deficit hyperactivity disorder is also provided as one embodiment of the present invention.

Further, as one embodiment of the present invention, there is provided the use of theanine for manufacturing a medicament for ameliorating attention-deficit hyperactivity disorder. According to the use, the attention-deficit hyperactivity disorder of the individual can be safely and effectively ameliorated without any risks of incidence of side effects.

In this embodiment, in general, as the effective dosage of theanine for obtaining the desired effects of the present invention, in a case of a human, the dosage is, for instance, preferably from 0.2 to 200 mg/kg weight, more preferably from 0.5 to 50 mg/kg weight per dose of one day. However, since there are some differences (age, sex, kinds and degree of symptoms, and the like) between individuals, the dosage of theanine in the present invention is not limited only to those ranges given above. The dosage of the theanine may be appropriately concretely adjusted individually so that the desired effects of the present invention can be obtained.

In order to administer theanine, there may be used theanine *per se*, or the composition of the present invention, preferably the food composition or the pharmaceutical composition. Also, the administration methods, the number of administration, the administration period, and the like are also not particularly limited. For instance, the theanine may be administered to the above-mentioned individual, preferably human within the above-mentioned effective dosage range at once or divided in plural times, preferably by oral administration. The theanine or the composition of the present invention can be administered on a daily basis so that prophylactic effects can be obtained.

The theanine used in the present invention has high safety. For instance, in an acute toxic test using a mouse, there are no cases of death with an oral administration at 5 g/kg, and there are found no abnormalities in the general states, weight and the like. Also, especially L-theanine is known as a main component of *umami* (tastiness) of the green tea, and is also used as a food additive giving *umami*, without the limitation of its added amount under the regulation for food hygiene. Moreover, contrary to the conventional drugs, since there is no side effect by theanine at all, the amelioration of attention-deficit hyperactivity disorder can be safely and effectively achieved according to the composition of the present invention.

Here, in the preparation of the composition used in Example 1, L-theanine [trade name: SUNTHEANINE, manufactured by Taiyo Kagaku Co., Ltd.] was used.

### Example 1

Five patients who were diagnosed attention-deficit hyperactivity disorder by doctors were administered with a composition containing L-theanine. The composition was prepared so that L-theanine was mixed with crystalline cellulose, sucrose fatty acid ester, reducing maltose, silicon dioxide, lactose and aspartame so as to have the content of L-theanine of 20% by weight, and tableting the mixture to prepare a tablet.

### (1) Patient 1

A 7-year-and-11-month-old boy (weight: 23.2 kg) having IQ 75: No abnormality during pregnancy or at birth has been especially found. The boy showed symptoms of hyperactivity, emotional instability, distraction and the like, such as not being able to sit still during a class, and not being able to listen to what others are telling him, so that he satisfied the above-mentioned [Criteria for Diagnosis] and was diagnosed ADHD. The boy was administered with L-theanine at 50 mg/day over a period of 8 weeks. After the termination of the administration, the above-mentioned symptoms substantially disappeared, with some of symptoms slightly yet remaining. The doctor in charge of the boy judged that the effect by the composition was "effective."

### (2) Patient 2

A 12-year-and-10-month-old girl (weight: 40.6 kg) having IQ 85: No abnormality during pregnancy or at birth has been especially found. The girl showed symptoms of insomnia, difficulty in concentration, emotional instability, and distraction, such as not being able to settle down, not being able to think before she acts, getting excited before others, not being able to listen to what others are telling her, disturbing playing of her friends, or the like, so that she satisfied the above-mentioned [Criteria for Diagnosis] and was diagnosed ADHD. Although the symptoms stabilized during the administration of Ritalin(methylphenidate), side effects were serious, so that the administration was stopped. The girl was administered with L-theanine at 200 mg/day over a period of 10 weeks. After the termination of the administration, the above-mentioned symptoms substantially disappeared, with some of symptoms slightly yet remaining. The doctor in charge of the girl judged that the effect by the composition was "effective.

### (3) Patient 3

A 4-year-and-6-month-old boy (weight: 18.2 kg) having IQ 80: No abnormality during pregnancy or at birth has been especially found. The boy showed symptoms of showing aggression, distraction, insomnia and violence, such as always interrupting conversations with others, not being able to easily fall asleep, and disturbing others, so that he satisfied the above-mentioned [Criteria for Diagnosis] and was diagnosed ADHD. The boy was administered with L-theanine at 100 mg/day together with Ritalin over a period of 1 week. As a result, the above-mentioned symptoms substantially disappeared, with some of symptoms slightly yet remaining.

### (4) Patient 4

A 15-year old boy (weight: 60.2 kg) having IQ 95: No abnormality during pregnancy or at birth has been especially found. The boy showed symptoms of getting excited in a person's presence, distraction, and violence so that he satisfied the above-mentioned [Criteria for Diagnosis] and was diagnosed ADHD. The boy was administered with L-theanine at 200 mg/day only once before he went to school. As a result, the boy could attend the classes without any problems. However, in the following day, the boy returned to the original state of symptoms, so that the maintenance by a single dosage administration could not be found.

### (5) Patient 5

A 22-year old male individual (weight: 60.6 kg) having IQ 70: No abnormality during pregnancy or at birth has been especially found. The male individual showed symptoms of hyperactivity, impulsive characteristic and distraction, such as always interrupting conversations with others, so that he satisfied the above-mentioned [Criteria for Diagnosis] and was diagnosed ADHD. The male was administered with L-theanine at 60 mg/day over a period of 1 week. After the termination of the administration, these symptoms were remarkably ameliorated, and the above-mentioned symptoms substantially disappeared, with some of symptoms slightly yet remaining.

It can be seen from the above examples that the composition of the present invention can ameliorate attention-deficit hyperactivity disorder safely and effectively.

### INDUSTRIAL APPLICABILITY

According to the present invention, there are provided a composition for use in ameliorating attention-deficit hyperactivity disorder and its use for manufacturing a medicament for ameliorating attention-deficit hyperactivity disorder. According to the composition and the use, since the attention-deficit hyperactivity disorder can be ameliorated safely and effectively, the composition and the use are useful for treatment, prevention or the like of the attention-deficit hyperactivity disorder.

## Claims

1. Use of theanine for manufacturing a medicament for ameliorating attention-deficit hyperactivity disorder.

2. Use of claim 1, wherein said theanine is present in an amount of from 0,00025 to 100% by weight based on the total weight of the medicament.

3. Use of claim 1, wherein said theanine is present in an amount of from 0,001 to 20% by weight based on the total weight of the medicament.

4. Use of claim 1, wherein said medicament further comprises a mineral.

5. Use of claim 1, wherein said theanine is purified theanine.

6. Use of claim 1, wherein said medicament is effective in treatment of symptoms of attention-deficit hyperactivity disorder, said symptoms selected from the group consisting of:
not being able to carefully pay attention to things; making mistakes due to lack of attention in schoolwork, vocational work or other activities; finding it difficult to maintain attention to assigned job or play; not seeming to be litening even when directly spoken to; not being able to carry out duties in schoolwork, daily miscellaneous trifles, or vocational work; not being able to orderly carry out assigned job or activity; avoiding or disliking to perform vocational or schoolwork that requires to maintain mental activities. or performing it unwillingly; losing things that are required for vocational work or activity; attention being distracted by stimuli from others; forgetting daily activities; busily moving hands or fidgetingly sitting; leaving ones seat or standing up in a classroom in a situation where one should not; running around or climbing over a certain object in an inappropriate situation; not being able to spend free time on quietly playing; constantly being active, moving about as if driven by a motor; over-talking; suddenly answering before a question is finished; not being able to wait in order; and disturbing others.

7. Theanine for use in ameliorating attention-deficit hyperactivity disorder.

8. Theanine for the use of claim 7, wherein said theanine is purified theanine.

9. Theanine for the use of claim 7 or 8, wherein theanine is effective in treatment of symptoms of attention-deficit hyperactivity disorder, said symptoms selected from the group consisting of:
not being able to carefully pay attention to things; making mistakes due to lack of attention in schoolwork, vocational work or other activities; finding it difficult to maintain attention to assigned job or play; not seeming to be litening even when directly spoken to; not being able to carry out duties in schoolwork, daily miscellaneous trifles, or vocational work; not being able to orderly carry out assigned job or activity; avoiding or disliking to perform vocational or schoolwork that requires to maintain mental activities. or performing it unwillingly; losing things that are required for vocational work or activity; attention being distracted by stimuli from others; forgetting daily activities; busily moving hands or fidgetingly sitting; leaving ones seat or standing up in a classroom in a situation where one should not; running around or climbing over a certain object in an inappropriate situation; not being able to spend free time on quietly playing; constantly being active, moving about as if driven by a motor; over-talking; suddenly answering before a question is finished; not being able to wait in order; and disturbing others.

## Patentansprüche

1. Verwendung von Theanin zur Herstellung eines Medikaments zur Verbesserung eines Aufmerksamkeits-Defizit-Syndroms mit Hyperaktivität.

2. Verwendung nach Anspruch 1, wobei das Theanin in einer Menge von 0,00025 bis 100 Gew.-%, bezogen auf das Gesamtgewicht des Medikaments, vorhanden ist.

3. Verwendung nach Anspruch 1, wobei das Theanin in einer Menge von 0.001 bis 20 Gew.-%, bezogen auf das Gesamtgewicht des Medikaments, vorhanden ist.

4. Verwendung nach Anspruch 1, wobei das Medikament weiter ein Mineral umfasst.

5. Verwendung nach Anspruch 1, wobei das Theanin gereinigtes Theanin ist.

6. Verwendung nach Anspruch 1, wobei das Medikament zur Behandlung von Symptomen des Aufmerksamkeits-Defizit-Syndroms mit Hyperaktivität wirksam ist, wobei die Symptome ausgewählt sind aus der Gruppe bestehend aus:
nicht in der Lage sein, Dingen sorgfältig Aufmerksamkeit zu schenken; Fehler machen aufgrund mangelnder Aufmerksamkeit bei Schulaufgaben, beruflicher Arbeit oder anderen Aktivitäten; es schwer finden, die Aufmerksamkeit auf eine bestimmte Arbeit oder ein bestimmtes Spiel gerichtet zu halten; anscheinend nicht zuhören, selbst bei direktem Angesprochenwerden; nicht in der Lage sein, die Aufgaben in einer Schulaufgabe, bei alltäglichen verschiedenen Kleinigkeiten oder bei der beruflichen Arbeit durchzuführen; nicht in der Lage sein, die zugewiesene Arbeit oder Aktivität ordentlich durchzuführen; eine berufliche oder Schulaufgabe, bei der gedacht werden muss, vermeiden oder ungern durchführen oder sie widerwillig durchführen; Dinge verlieren, die für die berufliche Arbeit oder Aktivität erforderlich sind; die Aufmerksamkeit wird durch Anreize von anderen abgelenkt; die täglichen Aktivitäten vergessen; geschäftig die Hände bewegen oder beim Sitzen herumzappeln; Verlassen des Sitzes oder Aufstehen im Klassenzimmer in einer Situation, in der man es nicht sollte; Herumlaufen oder Klettern über ein bestimmtes Objekt in einer unpassenden Situation; nicht in der Lage sein, die Freizeit mit ruhigem Spielen zu verbringen; ständig aktiv sein oder umherwandern wie von einem Motor angetrieben; übermäßiges Reden; plötzliches Antworten bevor eine Frage beendet ist; nicht in der Lage sein, in der Reihe zu warten; und andere stören.

7. Theanin zur Verwendung bei der Verbesserung eines Aufmerksamkeits-Defizit-Syndroms mit Hyperaktivität.

8. Theanin zur Verwendung nach Anspruch 7, wobei das Theanin gereinigtes Theanin ist.

9. Theanin zur Verwendung nach Anspruch 7 oder 8, wobei das Theanin zur Behandlung von Symptomen des Aufmerksamkeits-Defizit-Syndroms mit Hyperaktivität wirksam ist, wobei die Symptome ausgewählt sind aus der Gruppe bestehend aus:
nicht in der Lage sein, Dingen sorgfältig Aufmerksamkeit zu schenken; Fehler machen aufgrund mangelnder Aufmerksamkeit bei Schulaufgaben, beruflicher Arbeit oder anderen Aktivitäten; es schwer finden, die Aufmerksamkeit auf eine bestimmte Arbeit oder ein bestimmtes Spiel gerichtet zu halten; anscheinend nicht zuhören, selbst bei direktem Angesprochenwerden; nicht in der Lage sein, die Aufgaben in einer Schulaufgabe, bei alltäglichen verschiedenen Kleinigkeiten oder bei der beruflichen Arbeit durchzuführen; nicht in der Lage sein, die zugewiesene Arbeit oder Aktivität ordentlich durchzuführen; eine berufliche oder Schulaufgabe, bei der gedacht werden muss, vermeiden oder ungern durchführen oder sie widerwillig durchführen; Dinge verlieren, die für die berufliche Arbeit oder Aktivität erforderlich sind; die Aufmerksamkeit wird durch Anreize von anderen abgelenkt; die täglichen Aktivitäten vergessen; geschäftig die Hände bewegen oder beim Sitzen herumzappeln; Verlassen des Sitzes oder Aufstehen im Klassenzimmer in einer Situation, in der man es nicht sollte; Herumlaufen oder Klettern über ein bestimmtes Objekt in einer unpassenden Situation; nicht in der Lage sein, die Freizeit mit ruhigem Spielen zu verbringen; ständig aktiv sein oder umherwandern wie von einem Motor angetrieben; übermäßiges Reden; plötzliches Antworten bevor eine Frage beendet ist; nicht in der Lage sein, in der Reihe zu warten; und andere stören, ausgewählt sind.

## Revendications

1. Utilisation de la théanine pour la fabrication d'un médicament pour l'amélioration de troubles de manque d'attention / d'hyperactivité.

2. Utilisation selon la revendication 1, pour laquelle ladite théanine est présente en une quantité allant de 0,00025 à 100 % en masse en se fondant sur la masse totale du médicament.

3. Utilisation selon la revendication 1, pour laquelle ladite théanine est présente en une quantité allant de 0,001 à 20 % en masse en se fondant sur la masse totale du médicament.

4. Utilisation selon la revendication 1, pour laquelle ledit médicament comprend en outre un minéral.

5. Utilisation selon la revendication 1, pour laquelle ladite théanine est de la théanine purifiée.

6. Utilisation selon la revendication 1, pour laquelle ledit médicament est efficace pour le traitement de symptômes de troubles de manque d'attention / d'hyperactivité, lesdits symptômes étant sélectionnés parmi le groupe constitué par :
- ne pas être capable de prêter soigneusement attention à certaines choses ; faire des erreurs d'inattention au travail scolaire, professionnel ou à d'autres activités ; trouver difficile de maintenir son attention à des tâches ou jeux attribués ; ne pas sembler écouter même lorsque l'on s'adresse directement à vous ; ne pas être capable d'assumer des devoirs en ce qui concerne le travail scolaire, les tâches de la vie quotidienne ou le travail professionnel ; ne pas être capable d'exécuter méthodiquement une tâche ou une activité attribuée ; éviter ou détester effectuer un travail scolaire ou professionnel nécessitant des activités mentales soutenues ou l'effectuer à contrecoeur ; perdre des choses nécessaires à un travail ou une activité professionnelle ; avoir son attention distraite par des stimuli provenant d'autres personnes ; oublier des activités de la vie quotidienne ; avoir les mains agitées de manière affairée ou se tenir assis avec impatience ; quitter son siège ou se lever dans une salle de classe dans une situation où l'on ne devrait pas ; courir ça et là ou grimper sur un certain objet dans une situation inappropriée ; ne pas être capable d'occuper du temps libre à jouer tranquillement ; être constamment actif, être agité comme mû par un moteur ; parler trop ; répondre soudainement avant qu'une question ait fini d'être posée ; ne pas être capable d'attendre calmement ; et perturber d'autres personnes.

7. Théanine destinée à être utilisée pour améliorer des troubles de manque d'attention / d'hyperactivité.

8. Théanine pour l'utilisation selon la revendication 7, pour laquelle ladite théanine est de la théanine purifiée.

9. Théanine pour l'utilisation selon la revendication 7 ou 8, pour laquelle la théanine est efficace pour le traitement de symptômes de troubles de manque d'attention / d'hyperactivité, lesdits symptômes étant sélectionnés parmi le groupe constitué par :
- ne pas être capable de prêter soigneusement attention à certaines choses ; faire des erreurs d'inattention au travail scolaire, professionnel ou à d'autres activités ; trouver difficile de maintenir son attention à des tâches ou jeux attribués ; ne pas sembler écouter même lorsque l'on s'adresse directement à vous ; ne pas être capable d'assumer des devoirs en ce qui concerne le travail scolaire, les tâches de la vie quotidienne ou le travail professionnel ; ne pas être capable d'exécuter méthodiquement une tâche ou une activité attribuée ; éviter ou détester effectuer un travail scolaire ou professionnel nécessitant des activités mentales soutenues ou l'effectuer à contrecoeur ; perdre des choses nécessaires à un travail ou une activité professionnelle ; avoir son attention distraite par des stimuli provenant d'autres personnes ; oublier des activités de la vie quotidienne ; avoir les mains agitées de manière affairée ou se tenir assis avec impatience ; quitter son siège ou se lever dans une salle de classe dans une situation où l'on ne devrait pas ; courir ça et là ou grimper sur un certain objet dans une situation inappropriée ; ne pas être capable d'occuper du temps libre à jouer tranquillement ; être constamment actif, être agité comme mû par un moteur ; parler trop ; répondre soudainement avant qu'une question ait fini d'être posée ; ne pas être capable d'attendre calmement ; et perturber d'autres personnes.
